# EUROPEAN PATENT SPECIFICATION

(11) **EP 1 594 369 B1**
(45) Date of publication and mention of the grant of the patent: **21.04.2010**
(21) Application number: 04713096.8
(22) Date of filing: 20.02.2004
(51) Int. Cl.: A23L 1/218, C12N 1/20

(54) **CONTROL OF ENZYMATIC DEGRADATION OF GLUCOSINOLATES BY LACTIC ACID BACTERIUM**
KONTROLLE DES ENZYMATISCHEN ABBAUS VON GLUCOSINOLATEN DURCH MILCHSÄUREBAKTERIUM
REGULATION DE LA DEGRADATION ENZYMATIQUE DES GLUCOSINOLATES A L'AIDE D'UNE BACTERIE D'ACIDE LACTIQUE

(30) Priority: 21.02.2003 FI 20030258
(43) Date of publication of application: 16.11.2005
(73) Proprietor: Maa-Ja Elintarviketalouden Tutkimuskeskus, 31600 Jokioinen (FI); Bioferme OY, 20760 Piispanristi (FI)
(72) Inventor: MÄKI, Maarit, FI-30100 Forssa (FI); TOLONEN, Marja, FI-96460 Rovaniemi (FI); RAJANIEMI, Sirpa, FI-33710 Tampere (FI); PIHLAVA, Juha-Matti, FI-21290 Rusko (FI); RYHÄNEN, Eeva-Liisa, FI-00690 Helsinki (FI)
(74) Representative: Karvinen, Leena Maria
(86) International application number: PCT/FI2004/000076
(87) International publication number: WO 2004/073418

(56) References cited:
- US-A- 5 834 043
- DATABASE WPI Week198618, Derwent Publications Ltd., London, GB; Class D16, AN 1986-117198, XP002979273 & JP 61 058 574 A (KIKUNOKA SHUZO KK) 25 March 1986
- KYUNG K. H., ET AL.: 'Antimicrobial Activity of Sulfur Compounds Derived from Cabbage' JOURNAL OF FOOD PROTECTION vol. 60, no. 1, 1997, pages 67 - 71, XP002979274
- M. LLANOS PALOP, ET AL.: 'Degradation of sinigrin by Lactobacillus agilis strain R16' INTERNATIONAL JOURNAL OF FOOD MICROBIOLOGY vol. 26, 1995, pages 219 - 229, XP002979275
- KAROVICOVA J., ET AL.: 'The choice of strains of Lactobacillus species for the lactic acid fermentation of vegetable juices' EUR. FOOD RES. TECHNOL. vol. 210, 1999, pages 53 - 56, XP002979276

## Description

This invention relates to fermenting of plant-derived material containing glucosinolates with a starter containing a lactic acid bacterium, as well as to the lactic acid bacterium in question. Degradation products of the glucosinolates produced by the bacterium exhibit among other things antimicrobial properties, and may possess a health-promoting effect.

Preservation of vegetables by fermenting, utilizing lactic acid bacteria is an old process. The health-promoting effect of fermented products may at least partially be associated with intestinal function. The balancing effect of probiotic lactic acid bacteria on disturbed intestinal microbial flora, among other things, is well known (Gorbach, 1990), and probiotics are believed to lower serum cholesterol, improve the immune response and alleviate allergies. Furthermore, lactic acid bacteria decrease the activity of enzymes that activate carcinogenic compounds (Sanders, 1998, Ziemer and Gibson, 1998).

In studies utilizing cancer cells (*in vitro*) and experimental animals (*in vivo*), glucosinolates and their degradation products occurring in cabbage plants have been shown to exhibit an anticarcinogenic effect (Nastruzzi *et al.,* 1996, Leoni *et al.,* 1997, Zhang and Talalay, 1994). Degradation products of glucosinolates have been shown to inhibit the growth of *Escherichia coli* (Brabban and Edvards, 1995) and the yeasts *Saccharomyces cerevisiae, Torulopsis etchellsii, Hansenula mrakii, Pichia membranefaciens* (Kyung and Fleming, 1997).

Glucosinolates, of which more than 100 are currently known, are sulphur- and nitrogen- containing secondary plant compounds consisting of amino acid and sugar. The significance of glucosinolates is still unknown, but they have, among other things, been found to participate in the regulation of sulphur balance, germination and plant defence (Keskitalo, 2001). Glucosinolate-containing cruciferous plants can be used to clear up soil of herbicides. Glucosinolates are intrinsically non-toxic, but compounds formed from them may in high concentrations be detrimental to living organisms (Jaakkola, 2001). Benefits of these compounds as compared with pesticides are their spreading to the soil from plant roots and with plant litter and their rapid decomposition in the soil. They therefore are well suited for biological plant protection (Jaakkola, 2001).

Glucosinolates and their degradation products occurring in cabbage plants are important components affecting the sensory quality of foods. Certain degradation products of glucosinolates have been found to exhibit positive effects on health, rendering cabbage plants also attracting as potential materials for functional foods. The glucosinolate composition and concentration of cabbage plants varies with the species and varieties. Glucosinolate concentrations are affected by storage time and storage conditions. Glucosinolates are decomposed by the enzyme myrosinase, and methods of processing affect the amounts of degradation products (de Vos and Blijleven, 1988, Slominski and Campbell, 1989, Sørensen, 1990, Rosa and Heaney, 1993, Yen and Wei, 1993, Verkerk *et al*., 1997). In the preparation of sauerkraut, glucosinolates are decomposed within two weeks (Daxenbichler *el al*., 1980, Tolonen *et al.,* 2002).

The present invention relates to a starter that is applicable to fermenting of plant material, comprising the lactic acid bacterium *Lactobacillus* sp. 96/142 isolated from sauerkraut. The starter of the invention is mainly aimed at fermenting of cabbage plants.

The invention further relates to the above-mentioned *Lactobacillus* strain, which is likely to belong to the species *Lactobacillus sakei*.

The *Lactobacillus* sp. 96/142 strain of the invention produces from available sugars via fermentation among other things lactic acid, whereby growth of spoilage microorganisms is prevented. It additionally produces degradation products of glucosinolates possessing inhibitory effects on microbial growth and potential anticarcinogenic activity. Probiotic properties of the strain *Lactobacillus* sp. 96/142 were investigated in *in vitro* experiments and the strain was found to remain well viable at pH 4 but not at pH 2. The strain is thus expected to remain viable in the intestine, especially if it has protected itself with a self-produced exopolysaccharide or if it is microcapsulated. The health-promoting effect of the strain in the intestine is among other things based on its ability to produce from glucosinolates degradation products that may possess anticarcinogenic effects. In addition, production of lactic acid and lowering of intestinal pH are well-known properties balancing the intestinal flora.

The starter is added to the material to be fermented either as a freeze-dried powder or pre-cultured in vegetable juice. Along with the starter, other lactic acid bacterial strains can be added that affect the sensory and hygienic quality of the product but do not prevent the glucosinolate-degrading activity of the lactic acid bacterial strain of the invention. The culturing temperature should remain within the range 18 - 35 °C, as the strain of the invention is mesophilic.

The material to be fermented can be fresh or heat-treated plant material. By the heat treatment the plant's own myrosinase activity can almost completely be inactivated without a total destruction of glucosinolates (Ryhänen *et al*., 2001). The material is usually supplemented with salt, which by means of osmotic pressure removes fluid and nutrients from the plant cell tissue for microbial growth:

The present invention will be further described below by experiments and examples, and with reference to the attached drawings, in which
**Figures 1.1 and 1.2** present the experimental series 1 carried out with head cabbage during years 2000 - 2001:
   **1.1.** Degradation products of glucosinolates (response ratio × 100)/100 g f.wt. in fermented cabbage (average value and standard deviation, n = 3).
   **1.2** Degradation products of glucosinolates (µg/100 g f.wt.) (average value and standard deviation, n = 3).
**Figures 2.1 and 2.2** present fermentation experiments with cauliflower:
   **2.1** Degradation products of glucosinolates (response ratio × 100)/100 g f.wt. in fermented cauliflower.
   **2.2** Degradation products of glucosinolates in fermented cauliflower (µg/100 g f.wt.).
**Figures 3(a) to 3(f)** present the growth of indicator strains *E. coli, L. monocytogenes* and *C. lanibica* in fermented cabbage juice. In the figures, the change of the optical density of the cell mass is shown in absorbance (A₆₀₀) against time on a microtitre plate. *E. coli* (3 a, 3b); *L. monocytogenes* (3c, 3d) and *C. lambica* (3e, 3f). Juice dilutions 1:1 (3a, 3c, 3e) and 1:5 (3b, 3d, 3f). Initial concentrations of the indicator bacteria were 10³ cfu/ml. The average value and the standard deviation are depicted in the Figures (n = 3).

### Abbreviations used:

| | |
|---|---|
| ITC | isothiocyanate |
| d.m. | dry matter |
| cfu | colony forming units |
| f.wt. | fresh weight |

### Isolation and characterization of the lactic acid bacterial strain of the invention

From a spontaneous fermentation of sauerkraut 236 lactic acid bacterial strains were isolated from MRS medium (De Man *et al.,* 1960). *Lactobacillus* sp. 96/142 was isolated 6 d after start of preservation from the juice that had separated from the sauerkraut. The strain was subjected to physiological and biochemical tests: gas formation from glucose, hydrolysis of arginine, configuration of lactic acid, and the API test (fermentation of sugars, Table 1).

**Table 1. Fermentation of sugars and sugar alcohols by the strain of the invention.**

| | *Lactobacillus* sp. 96/142 (anal. 30.10.2002) |
|---|---|
| L-arabinose | + |
| Ribose | + |
| D-xylose | - |
| Galactose | + |
| D-glucose | + |
| D-fructose | + |
| D-mannose | + |
| Rhamnose | (+) |
| Mannitol | - |
| Sorbitol | - |
| α-methyl-D-glucoside | - |
| N-acetyl-glucosamine | + |
| Amygdaline | - |
| Arbutine | - |
| Esculine | + |
| Salicine | (+) |
| Cellobiose | + |
| Maltose | + |
| Lactose | (+) |
| Melibiose | + |
| Sucrose | + |
| Trehalose | + |
| Melezitose | - |
| Raffinose | - |
| β-gentibiose | + |
| D-turanose | - |
| D-arabitol | - |
| Gluconate | (+) |

In addition to these tests the strain was identified using ribotyping (VTT Biotechnology), and it was found likely to be *Lactobacillus sakei*. On Rogosa SL medium *Lactobacillus* sp. 96/142 exclusively produced the L(+)-isomer of lactic acid. In sauerkraut it, however, produced both isomers of lactic acid in equal amounts. During fermentation in sauerkraut the strain produced exopolysaccharide in abundance. Exopolysaccharide formation has also been noted with other strains of *L. sakei* and they can be utilized to improve food structure (Degeest *et al.,* 2001). The strain would be well suited for improving the structure of liquid foods prepared from vegetables. The applicability of strain *Lactobacillus* sp. 96/142 as a probiotic was investigated in *in vitro* tests. *Lactobacillus* sp. 96/142 was deposited on 22 November 2002 according to the Budapest treaty in the DSMZ depository (Deutsche Sammlung von Mikroorganismen und Zellkulturen, GmbH) under the accession number DSM 15298.

### Experiments on the properties of products prepared with the bacterial strain of the invention, on the applicability of the strain to fermenting, and on its effect on the degradation products of glucosinolates.

### Experiments with head cabbage

### Experimental series 1 (2000 - 2001)

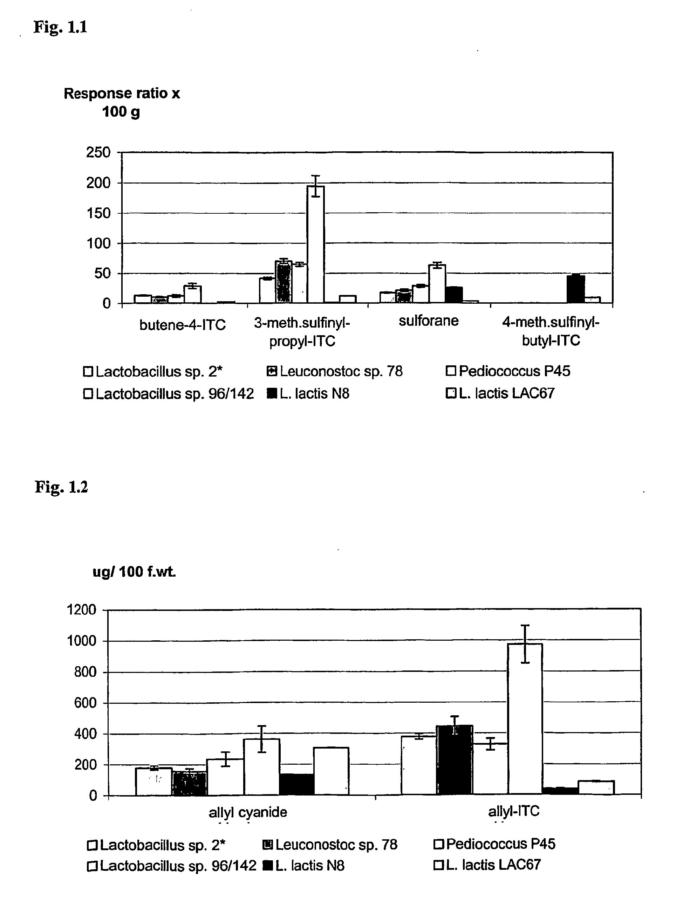

Fermenting of head cabbage has been studied with lactic acid bacterial pure cultures. Analyses were carried out with the fermented cabbages for acid formation, microbes and sensory quality (Table 2), and with the extract obtained from sauerkraut with an organic solvent for degradation products of glucosinolates (Tolonen *el al*., 2002) (Figures 1.1 and 1.2).

**Table 2. Chemical composition of sauerkrauts fermented either with pure cultures or spontaneously, and numbers of lactic acid bacteria (cfu/ml) after 14 days of fermentation.**

| Starter strain used in fermentation *(n=3) | Glucose ⁽¹⁾ (g l⁻¹) | Titratable acidity (%) | Lactic acid (g l⁻¹) | Acetic acid (g l⁻¹) | Lactic acid bacteria |
|---|---|---|---|---|---|
| Raw material (t = 0 h) ⁽²⁾ (n = 6) | 11 ± 1.0 | 0.1 | n.d. | n.d. | 10⁴ - 10⁵ |
| *Lactobacillus* sp. 2* (DSM 14485) | 12.5 ± 0.2 | 1.0 ± < 0.1 | 11.5 ± 0.3 | 0.5 ± < 0.1 | 2.5 × 10⁹ |
| **Leuconostoc* sp. 78 (DSM 14486) | 12.3 ± 0.5 | 1.1 ± < 0.1 | 4.8 ± 0.2 | 2.7 ± < 0.1 | 3.8 × 10⁹ |
| **Pediococcus* sp. P45 (DSM 14488) | 17.8 ± 0.1 | 0.7 ± < 0.1 | 5.2 ± 0.1 | 0.1 ± < 0.1 | 2.5 × 10⁷ |
| **Lactobacillus* sp. 96/142 | 9.2 ± 0.3 | 0.5 ± < 0.1 | 6.5 ± 0.2 | 0.3 ± < 0.1 | 9.2 × 10⁹ |
| **L. lactis* N8 | 23.3 ± 0.4 | 0.4 ± < 0.1 | 4.4 ± 0.1 | 0.1 ± < 0.1 | 8.1 × 10⁸ |
| **L. lactis* LAC67 | 23.4 ± 0.5 | 0.8 ± < 0.1 | 5.3 ± 0.2 | 0.8 ± < 0.1 | 8.2 × 10⁸ |
| Spontaneous fermentation (n = 6) | n.d. | 0.8 ± 0.3 | n.d. | n.d. | 10⁵ - 10⁸ |

| | | | | | |
|---|---|---|---|---|---|
| (1) Concentration of free glucose (in the juice) (2) Values determined immediately after addition of the starter n.d. = not determined | | | | | |

Cabbages fermented with the starter were of equal quality, whereas the quality waried among spontaneously fermented cabbages. In the raw material the number of yeasts and moulds ranged from 100 to 1000 cfu/ml and that of coliform enterobacteria from 0 to 100 cfu/ml. Their growth was prevented when the sauerkaut was fermented with pure cultures of lactic acid bacteria. In the spontaneously fermented sauerkraut the numbers of yeasts and moulds ranged from 0 to 10 cfu/ml and those of coliform enterobacteria from 0 to 1000 cfu/ml. The highest sensory quality was obtained with *Leuconostoc* sp. 78 (DSM 14486) and with *Lactobacillus* sp. 2* (DSM 14485).

The glucosinolate concentrations of head cabbage (variety Lennox) were determined during four years (Table 3).

**Table 3. Concentrations of glucosinolates in cabbage during years 1998 - 2000 (Tolonen et al., 2002) and 2001 (unpublished) (µmol/g d.m.).**

| Glucosinolate | 1998^{a} | 1999 | 2000 | 2001^{d} | 2001^{e} |
|---|---|---|---|---|---|
| | Average (range)^{b} | Average (range)^{c} | Average (range)^{c} | Average (range)^{b} | Average (range)^{b} |
| Sinigrin | 1.43 (0.86-2.03) | 8.5 (7.33-9.79) | 5.47 (5.27-5.92) | 4.6 (3.4-5.9) | 7.5 (7.3-7.9) |
| Glucoiberin | 1.06 (0.73-1.29) | 5.1 (4.18-6.28) | 4.6 (4.24-5.16) | 4.5 (3.9-5.3) | 7.1 (6.6-8.0) |
| Glucobrassicin | 0.49 (0.29-0.79) | 0.46 (0.43-0.47) | 1.7 (1.61-1.86) | 1.3 (1.2-1.4) | 1.5 (1.0-1.9) |
| Progoitrin | 0.18 (0-0.37) | 1.32 (1.22-1.37) | 1.33 (1.25-1.46) | <0.1 | <0.1 |
| Glucorafanin | 0.2 (0-0.31) | 0.94 (0.76-1.28) | 1.28 (1.17-1.46) | - | - |
| Gluconapin | 0.2 (0-2.24) | 0.82 (0.71-0.93) | 0.63 (0.54-0.74) | 0.2 (0.2-0.3) | 0.5 (0.4-0.7) |
| 4-OH-glucobrassicin | <0.1 (0-0.08) | 0.13 (0.08-0.15) | 0.19 (0.13-0.24) | 0.1 (0.0-0.1) | 0.1 (0.1-0.2) |
| 4-MeO-glucobrassicin | 0.11 (0.07-0.16) | 0.55 (0.45-0.66) | 0.35 (0.31-0.38) | 0.2 | 0.4 (0.3-0.4) |
| neoglukobrassicin | - | - | - | 0.1 (0.0-0.1) | 0.1 (0.0-0.1) |
| Total | 3.71 | 17.82 | 15.55 | 11.0 | 17.3 |

| | | | | | |
|---|---|---|---|---|---|
| a) Cabbage stored (+ 2 °C) for 8 months before analysis and fermentation b) n=3 c) n=4 d) Normal nitrogen fertilization during growing season = A e) No nitrogen fertilization during growing season = B | | | | | |

Volatile degradation products of glucosinolates in sauerkrauts fermented with pure cultures are shown in Figures 1.1 and 1.2. The total concentration of degradation products was 2 to 3 times higher in the sauerkraut fermented with strain *Lactobacillus* sp: 96/142 as compared with the others. Of the individual degradation: products, especially the concentrations of 3-methylsulfinylpropyl-ITC and allyl-ITC were multifold with strain *Lactobacillus* sp. 96/142. Concentrations of indole carbinol and indole acetonitrile were below detection limit (10 - 50 µg/100g f.wt.) in all fermentations. Indole glucosinolates may occur as ascorbigen, but they were not determinend. The amount of degradation products of glucosinolates is related with the amount of starting raw material. Concentration of goitrin was high in all treatments (500 - 1100 µg/100 g f.wt.).

### Experimental series 2 (2001 - 2002).

Head cabbages (variety Lennox) had been grown at MTT Horticulture with nitrogen supplementation (A) and without nitrogen supplementation (B) during the growing season. The cabbages were fermented by adding the strains *Leuconostoc* sp. 78, *Lactobacillus* sp. 2* and *Lactobacillus* sp. 96/142 at 10⁵ - 10⁶ cfu/g. The cabbages were fermented for 14 d. pH and amount of glucose were higher in the sauerkraut prepared from cabbages grown without nitrogen supplementation than those of cabbages fertilized during the growing season, and the pH and sugar of sauerkrauts prepared with strain *Lactobacillus* sp. 96/142 were higher than those of cabbages prepared with *Lactobacillus* sp. 2*, which was used for comparison (Table 4). Numbers of lactic acid bacteria were 10⁷ - 10⁸ cfu/ml. Growth of coliform enterobacteria was prevented. Based on the results, the optimum pH range of strain *Lactobacillus* sp. 96/142 was higher than that of strain *Lactobacillus* sp. 2* and the growth of this strain was prevented upon decrease of pH to approx. 3.9.

**Table 4. Average values and standard deviations of glucose concentrations and pH values after 14 days of preservation per segment (n = 5). A = nitrogen supplementation during the growing season, B = no nitrogen supplementation.**

| Cabbage | Glucose g/l | | pH | |
|---|---|---|---|---|
| | *Lactobacillus* sp. 96/142 | *Lactobacillus* sp. 2* | *Lactobacillus* sp. 96/142 | *Lactobacillus* sp. 2* |
| A | 25.6 ± 2.5 | 18.3 ± 3.5 | 3.90 ± 0.05 | 3.62 ± 0,05 |
| B | 28.5 ± 1.0 | 26.0 ± 3.26 | 3.92 ± 0.02 | 3.64 ± 0.01 |

Cabbages of the autumn's sauerkraut experiment were evaluated in two evaluation sessions, in which cabbage A and cabbage B and the bacterial strains *Lactobacillus* sp. 2* vs. *Lactobacillus* sp. 96/142 and *Lactobacillus* sp. 2* vs. *Leuconostoc* sp. 78 were compared in pairs. Cabbages A were better in appearance and smell. The most pleasant one turned out to be cabbage A/ *L. plantanum* 2* in both tests. Among the cabbages B *Lactobacillus* sp. 96/142 was less acidic and aromatic than *Lactobacillus* sp. 2* (P=0.0011 and P=0.033, respectively, Kruskall-Wallis test).

### The cauliflower experiment

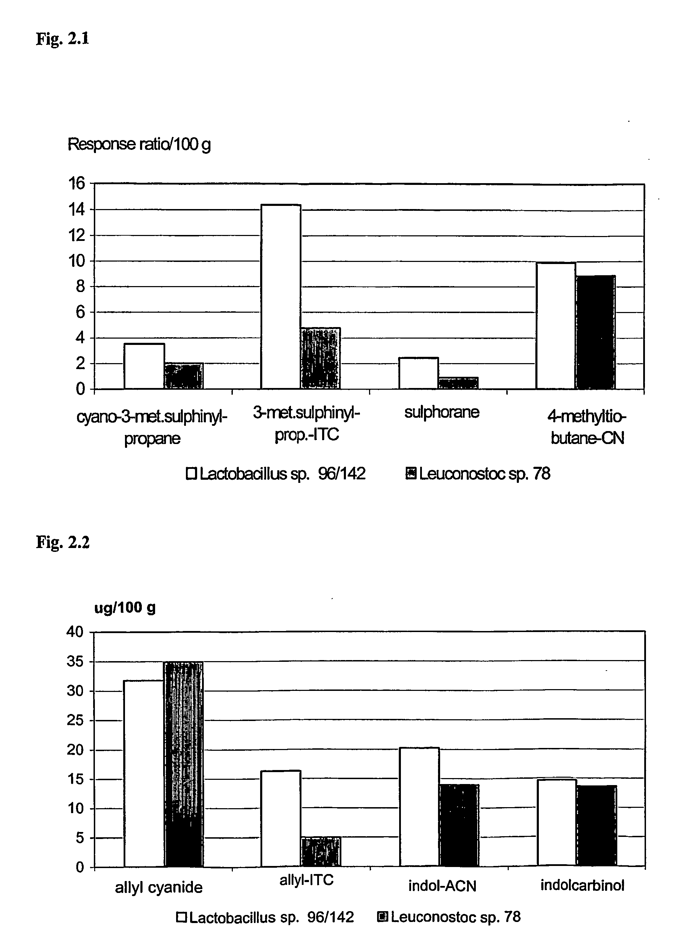

A preservation experiment with commercial cauliflower was carried out on 24.8.2001 with *Lactobacillus* sp. 96/142 and *Leuconostoc* sp. 78. Grated cauliflower was supplemented with 1 % of table salt and starter at 10⁶ cfu/g and fermenting was continued for 3 d. In both treatments the growth of coliform enterobacteria was prevented in three days and the pH decreased to 3.9 - 4.0. *Lactobacillus* sp. 96/142 produced an aromatic and atypical taste to the cauliflower, deviating form the savoury and fresh cauliflower prepared with *Leuconostoc* sp. 78. The amounts of glucosinolate were considerably smaller than those of head cabbage (Table 5), and the amounts of their volatile degradation products were correspondingly lower (Figures 2.1 and 2.2). Also in this experiment *Lactobacillus* sp. 96/142 produced considerably more 3-methylsulfinylpropyl-ITC and allyl-ITC than *Leuconostoc* sp. 78. In addition, *Lactobacillus* sp. 96/142 produced 3-OH-2-butanone in abundance, whereas *Leuconostoc* sp. 78 did not produce it at all.

**Table 5. Glucosinolate concentrations of commercial cauliflower**

| Glucosinolate | µmol/g d.m. |
|---|---|
| Sinigrin | 1.9 |
| Glucoiberin | 1.4 |
| Glucobrassicin | 2.2 |
| Progoitrin | <0.1 |
| Glucorafanin | - |
| Gluconapin | - |
| 4-OH-glucobrassicin | 0.8 |
| 4-MeO-glucobrassicin | 0.1 |
| Neoglucobrassicin | 0.5 |
| Total | 6.9 |

### Tests for antimicrobial activity

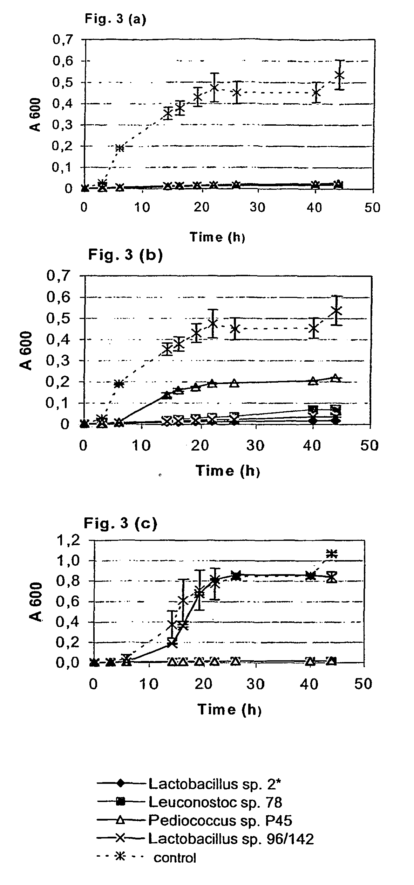

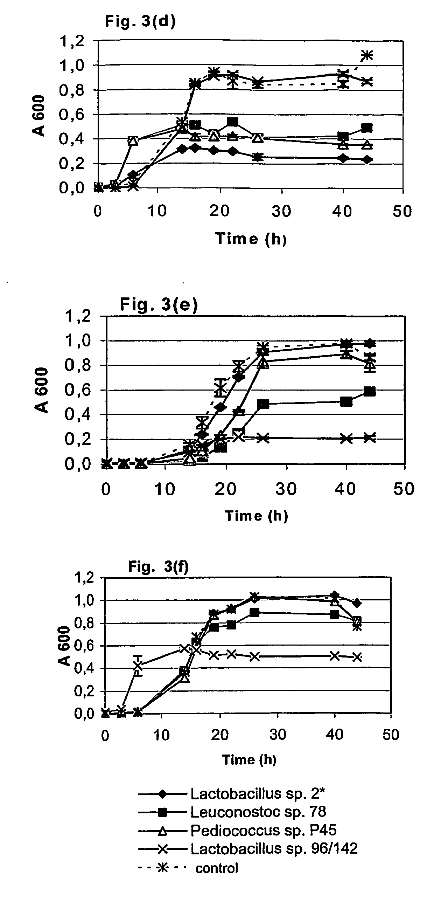

It this test the antimicrobial activity of cabbage juices fermented with strains *Lactobacillus* sp. 2*, *Leuconostoc* sp. 78, *Pediococcus* sp. P45 and *Lactobacillus* sp. 96/142 was tested on the growth of indicator strains *Listeria monocytogenes, Micrococcus luteus, Escherichia coli* and *Candida lambica* (Figures 3a to 3f). Growth was tested at different juice dilutions: 1:1, 1:5 and 1:10. The pH value of the sauerkraut juice was adjusted to 6.5, whereby the effect of acids formed in the fermentation on the growth of indicator strains was eliminated. Cabbage juice alone (pressed at time of fermentation t = 0 h) did not inhibit growth of the indicator strains. *M luteus* grew weakly in each cabbage juice and also in the control. All strains effectively inhibited the growth of *E. coli* at dilutions 1:1 and 1:5. *Lactobacillus* sp. 96/142 differed from the other plant-derived strains in that the juice fermented with it did not inhibit the growth of the bacterium *L. monocytogenes,* but effectively inhibited the growth of the yeast *C. lambica* (Figure 3e and 3f). Juice produced with the strain *Leuconostoc* sp. 78 inhibited the growth of the yeast *C. lambica* to some extent. At all juice dilutions of 1:10 the indicator strain grew as in the control; no inhibitory effect was noted. Based on the analysis results of degradation products of glucosinolates *Lactobacillus* sp. 96/142 in comparison with the other strains tested produces more allyl isothiocyanate, which has been found to exhibit strong antimicrobial activity.

As compared with sodium benzoate, the effect is 20 to 100-fold higher within a pH range of 5 - 7 (Kyung and Fleming, 1997).

### Sensory experiments

The sensory quality of sauerkrauts preserved with pure cultures and with mixtures of strains has been compared in these tests. Based on the results *Lactobacillus* sp. 96/142 does not cause distaste to sauerkraut, but the final product is less acidic and less aromatic than products prepared with strains *Lactobacillus* sp. 2*, *Leuconostoc* sp. 78 and *Pediococcus* sp. P45. By combining strains in different proportions it was found that by supplementing strain *Lactobacillus* sp. 96/142 with 10 % of one of the above-mentioned strains the products are good-tasting and indistinguishable from products preserved with added pure strains.

### Deposited microorganisms

The following microorganism was deposited according to the Budapest treaty in the DSMZ depository (Deutsche Sammlung von Mikroorganismen und Zellkulturen, GmbH, Mascheroder Weg 1b, D-38124 Braunschweig, Germany)

| **Microorganism** | **Accession number** | **Date of deposition** |
|---|---|---|
| *Lactobacillus* sp. 96/142 | DSM 15298 | 22 November 2002 |

### References:

Brabban, A.D. and Edwards, C. 1995. The effects of glucosinolates and their hydrolysis products on microbial growth. J. Appl. Bact. 79:171-177.
Daxenbichler, M.E., VanEtten, C.H. and Williams, P.H. 1980. Glucosinolate products in commercial sauerkraut. Journal of Agricultural and Food Chemistry 28:809-811.
Degeest, B., Janssens, B and De Vuyst, L. 2001. Exopolysaccharide (EPS) biosynthesis by Lactobacillus sakei 0-1: production kinetics, enzyme activities and EPS yields. J. Appl. Microbiol. 91:470-477.
Gorbach. 1990. Lactic acid bacteria and human health. Ann. Med. 22:37-41.
Jaakkola, S. 2001. Glukosinolaatit ja niiden hajoamistuotteet kasvinsuojelussa. In: Kasviperäiset biomolekyylit - glukosinolaatit (ed. Hyvärinen, H.) Maatalouden tutkimuskeskuksenjulkaisuja, Sarja A 90. ss. 32- 57.
Keskitalo, M. 2001. Glukosinolaattien biokemia ja esiintymiseen vaikuttavat kasvutekijät. In: Kasviperäiset biomolekyylit - glukosinolaatit (ed. Hyvärinen, H.) Maatalouden tutkimuskeskuksen julkaisuja, Sarja A 90. ss. 10 - 31.
Kyung, K.H. and Fleming, H.P. 1997. Antimicrobial activity of sulfur componds derived from cabbage. J. Food Prot. 60:67-71.
Leoni, O., Iori, R., Palmieri, S., Esposito, E., Menegatti, E., Cortesi, R. and Nastruzzi, C. 1997. Myrosinase-generated isothiocyanate from glucosinolates: isolation, characterization and in vitro antiproliferative studies. Bio-organic & Medicinal Chemistry 5 (9):1799-1806.
DeMan, J.C., Rogosa, M. and Sharpe, M.E. 1960. Medium for the cultivation of lactobacilli. J. Appl. Bact. 23:130-135.
Nastruzzi, C., Cortesi, R., Esposito, E., Menegatti, E., Leoni, O., Iori, R. and Palmieri, S. 1996. In vitro cytotoxic activity of some glucosinolate-derived products generated by myrosinase hydrolysis. J. Agric. Food Chemistry 44:1014-1021.
Rosa, E.A.S. and Heaney, K.H. (1993) The effect of cooking and processing on the glucosinolate content: studies on four varietes of portuguese cabbage and hybrid white cabbage. J. Sci Food Agric. 62:259-265.
Ryhänen, E-L., Tolonen, M. and Taipale, M. 2001. Giukosinolaatit ja niiden hajoamistuotteet elintarvikkeissa. In: Kasviperaiset biomolekyylit - glukosinolaatit (ed. Hyvärinen, H.) Maatalouden tutkimuskeskuksen julkaisuja, Sarja A 90. ss. 58-67.
Sanders, M.E. 1998. Overview of functional foods: Emphasis on probiotic bacteria. International Dairy Journal 8:341-347.
Tolonen, M., Taipale, B., Viander, B., Pihlava, J-M., Korhonen, H. and Ryhänen, E-L. (2002). Plant-derived biomolecules in cabbage. J. Agric. Food Chem. 50 (23) 6798-6803, doi:10.1021/jf0 109017.
De Vos, R. H. and Blijleven, W. G. H. (1988) The effect of processing conditions on glucosinolates in cruciferous vegetables. Z. Lebensm. Unters. Forsch. 187:525-529.
Zhang, Y. and Talalay, P. 1994. Anticarcinogenic activities of organic isothiocyanates: chemistry and mechanism. Cancer Research (Suppl.) 54:1976-1981).
Ziemer, C.J., and Gibson, G.R. (1998). An overview of probiotics, prebiotics and synbiotics in the functional food concept: perspectives and future strategies. International Dairy Journal 8: 473-479.

## Claims

1. Starter applicable to fermenting of vegetable material, **characterized in that** it comprises the bacterial strain *Lactobacillus* sp. 96/142 having the deposit accession number DSM 15298.

2. Pure culture of the bacterial strain *Lactobacillus* sp. 96/142 (DSM 15298).

3. Use of bacterial strain *Lactobacillus* sp. 96/142 (DSM 15298) in the starter applicable to fermenting of vegetable material.

4. Use of bacterial strain *Lactobacillus* sp. 96/142 (DSM 15298) for degrading glucosin.o-lates contained in vegetable material.

5. Use of bacterial strain *Lactobacillus* sp. 96/142 (DSM 15298) for producing antimicrobial compounds from glucosinolates.

6. A method for degrading glucosinolates contained in vegetable material, comprising fermenting said vegetable material with a starter comprising the bacterial strain *Lactobacillus* sp. 96/142 having the deposit accession number DSM 15298.

7. A method for the production of antimicrobial compounds from glucosinolates contained in vegetable material, comprising fermenting said vegetable material with a starter comprising the bacterial strain *Lactobacillus* sp. 96/142 having the deposit accession number DSM 15298.

## Patentansprüche

1. Starter, der zur Fermentation von pflanzlichem Material geeignet ist, **dadurch gekennzeichnet, dass** er den Bakterienstamm *Lactobacillus sp.* 96/142 mit der Hinterlegungsnummer DSM 15298 umfasst.

2. Reinkultur des Bakterienstamms *Lactobacillus sp.* 96/142 (DSM 15298).

3. Verwendung des Bakterienstamms *Lactobacillus sp*. 96/142 (DSM 15298) in dem Starter, der zur Fermentation von pflanzlichem Material geeignet ist.

4. Verwendung des Bakterienstamms *Lactobacillus sp.* 96/142 (DSM 15298) zum Abbau von Glucosinolaten, die in pflanzlichem Material enthalten sind.

5. Verwendung des Bakterienstamms *Lactobacillus sp.* 96/142 (DSM 15298) zur Herstellung antimikrobieller Verbindungen aus Glucosinolaten.

6. Verfahren zum Abbau von Glucosinolaten, die in pflanzlichem Material enthalten sind, umfassend die Fermentation des pflanzlichen Materials mit einem Starter, der den Bakterienstamm *Lactobacillus sp.* 96/142 mit der Hinterlegungsnummer DSM 15298 umfasst.

7. Verfahren zur Herstellung von antimikrobiellen Verbindungen aus Glucosinolaten, die in pflanzlichem Material enthalten sind, umfassend die Fermentation des pflanzlichen Materials mit einem Starter, der den Bakterienstamm *Lactobacillus sp.* 96/142 mit der Hinterlegungsnummer DSM 15298 umfasst.

## Revendications

1. Culture starter utilisable pour la fermentation d'une matière végétale, **caractérisée en ce qu'**elle comprend la souche bactérienne *Lactobacillus* sp. 96/142 ayant le numéro de dépôt DSM 15298.

2. Culture pure de la souche bactérienne *Lactobacillus* sp. 96/142 (DSM 15298).

3. Utilisation de la souche bactérienne *Lactobacillus* sp. 96/142 (DSM 15298) dans la culture starter utilisable pour la fermentation d'une matière végétale.

4. Utilisation de la souche bactérienne *Lactobacillus* sp. 96/142 (DSM 15298) pour dégrader les glucosinolates contenus dans une matière végétale.

5. Utilisation de la souche bactérienne *Lactobacillus* sp. 96/142 (DSM 15298) pour produire des composés antimicrobiens à partir de glucosinolates.

6. Procédé de dégradation de glucosinolates contenus dans une matière végétale, comprenant la fermentation de ladite matière végétale avec une culture starter comprenant la souche bactérienne *Lactobacillus* sp. 96/142 ayant le numéro de dépôt DSM 15298.

7. Procédé de production de composés antimicrobiens à partir de glucosinolates contenus dans une matière végétale, comprenant la fermentation de ladite matière végétale avec une culture starter comprenant la souche bactérienne *Lactobacillus* sp. 96/142 ayant le numéro de dépôt DSM 15298.
